# EUROPEAN PATENT SPECIFICATION

(11) **EP 1 294 428 B1**
(45) Date of publication and mention of the grant of the patent: **10.05.2017**
(21) Application number: 01947705.8
(22) Date of filing: 29.06.2001
(51) Int. Cl.: A61M 16/16, A61M 16/10, A61M 13/00

(54) **APPARATUS FOR HUMIDIFICATION AND WARMING OF AIR**
VORRICHTUNG ZUM BEFEUCHTEN UND ERWÄRMEN VON LUFT
APPAREIL PERMETTANT D'HUMIDIFIER ET DE RÉCHAUFFER L'AIR

(30) Priority: 30.06.2000 US 215442 P
(43) Date of publication of application: 26.03.2003
(60) Divisional application: 10012593.9
(73) Proprietor: Northgate Technologies Incorporated, Elgin, IL 60123 (US)
(72) Inventor: MANTELL, Robert, A., Arlington Heights, IL 60004 (US); MANZIE, Peter, A., Burwyn, IL 60402 (US)
(74) Representative: Grünecker Patent- und Rechtsanwälte PartG mbB
(86) International application number: PCT/IB2001/001162
(87) International publication number: WO 2002/000284

(56) References cited:
- EP-A- 0 567 158
- EP-A- 1 005 878
- WO-A-91/19527
- DE-A- 19 923 297
- DE-A1- 2 810 325
- FR-A- 2 276 065
- FR-A- 2 636 845
- GB-A- 2 173 108
- US-A- 3 659 604
- US-A- 3 954 920
- US-A- 4 054 622
- US-A- 4 110 419
- US-A- 4 225 542
- US-A- 4 657 713
- US-A- 4 715 998
- US-A- 4 921 642
- US-A- 5 195 515

## Description

### BACKGROUND OF THE INVENTION

### Field of the Invention

The present invention relates to an apparatus used to humidify and/or warm a gas prior to its use in a surgical or other medical procedure.

### Discussion of Related Art

Many medical and surgical procedures require the supply to a patient of warmed and/or humidified gas at constant high flow rates. Ideally, the flow rate should be approximately 20 liters per minute, the relative humidity should be approximately 80 to 100 percent, and the temperature approximately 90 to 105 degrees Fahrenheit. Most prior art devices cannot meet or exceed these ideal characteristics. The flow rate of many prior devices is well below 20 liters per minute. Commonly, the flow rate of prior devices has been generally 12 to 14 liters per minute. Most of these devices generally operate by forcing the gas through the humidification material, thereby requiring a high degree of pressure. This increased pressure reduces the flow rate of the gas even further.

DE 199 23 297 A describes a fluid treatment device which comprises a housing and a fluid treatment element, eg a filter, located in the housing to treat fluid flowing through the housing. The housing has an inlet and an outlet port and is of elongate shape divided along its length into two parts, each part having a set of triangular tooth formations extending along opposite sides that mate with one another. The fluid treatment element is pleated across its width in a zigzag fashion. The device may be used as a catheter mount for medical respiration systems with a patient end fitting and a machine end fitting. Alternatively the fluid treatment device may be used for humidifying air flowing to or from a patient, in which case the fluid treatment element may be an HME (heat and moisture exchange) element.

FR 2 636 845 A describes a humidifier of small gases passageway volume of the order of 50 ml gases space) which has a heater within a water containing envelope having a microporous wall common to the water space within the envelope end the gases space, the microporous wall is permeable to water vapour but not liquid water and the envelope being reinforced by a support which also directs the flow of gases over the surface of the envelope. The heater is wound as a flat spiral to avoid the effect of gases or water vapour bubbles on heater performance temperature.

EP 0 567 158 A discloses a humidifier for providing humidified gases to a patient. The humidifier has a gases volume of the order of 60mls. A microporous water compartment (16) is provided perpendicular to the gases flow to ensure humidification of the gases. Also, a combined temperature probe and heater supply connector is provided which only supplies energy to the heater in the water compartment when the gases temperature sensor is appropriately positioned in the humidifier gases passageway. This reduces the risk of gases at excessively high temperatures being supplied to the patient.

US 3 659 604 A discloses a humidifying means which has a contained for water which is removably from a source of heat, with the container being provided with a cover defining an air space with an inlet for gases and an outlet for gases and water vapor, the whole is removable for sterilization or other purposes. Temperature control means are provided externally of the container to control the temperature of the water in the container.

GB 2 173 108 A discloses a humidifier which includes a chamber having an inlet and an outlet. The chamber is releasably attached to a heater unit including a heater and a heat sensor. When the chamber and the heater unit are assembled together the heater is in thermal contact with a heat transfer surface of the chamber at or adjacent the inlet port while the temperature sensor is in thermal contact with the heat transfer surface at or adjacent the outlet port.

WO 91 19527 discloses a device for compensating for the moisture and heat losses occurring in artificial noses to be incorporated in the duct for supplying air and/or gasses to a patient and which is composed of a chamber used as actual artificial nose wherein a hygroscopic material serving as a buffer is provided, with means to maintain the in- an exhaled between the artificial nose and the patient at the required temperature level.

FR 2 276 065 A discloses a breathing air humidifier for respirator devices comprising an inspiration conduit which is connected to the mouth of the user of the respirator and which has a section or portion with a foil surface of a material which is impervious to water but pervious to water vapor. A water chamber surrounds the foil surface and water which is warmed is delivered to the water chamber from a supply tank located above the chamber which carries a heater for warming the water to a preselected temperature.

EP 1 005 878 A discloses a system for monitoring changes in a patient's body resources during breathing aided by a breathing apparatus which comprises means adapted to determine the difference between corresponding values of either one or both of moisture content and temperature of each of an inspiration gas and an expiration gas within the breathing apparatusas determined by moisture and temperature sensor elements within a common inspiration/expiration gas flow path; and processing means adapted to process the determined difference to provide an output related to the change in the associated body resource useable in the control of for example, an inspiration gas pre-conditioning unit to minimize the determine difference.

US 4 054 622 A discloses a self-contained disposable unit for converting a liquid to aerosol or for humidifying a gas includes gas inlet means and an enclosed container having a liquid supply reservoir and an outlet for the aerosol or for the humidified gas. A nozzle assembly for producing aerosol extends into the container above the liquid supply reservoir and a dual purpose conduit in communication with the liquid supply reservoir either carries liquid from the reservoir to the nozzle assembly during nebulization or passes gas into the liquid in the reservoir during humidification.

US 5 195 515 A discloses a heated cartridge humidifer for use with a collapsible water supply container and a heating device the humidifier adapted for heating and humidifying a breathable gas to be inhaled by a patient. The humidifier includes a humidifier housing having an open upper end. A cap portion sealingly engages the open upper end of the humidifier housing to define a humidifier chamber, and has a gas inlet port, a gas delivery port, and a water delivery port adapted to receive water from the container, all of the ports being in fluid communication with the humidifier housing.

US 4 715 998 A discloses a humidifying module which is releasably attachable to heater unit to form an apparatus for delivering humidified gas to a patient and includes a substantially flat wick which absorbs water without any substantial change in its surface area. The wick is contained within a chamber defined by a substantially flat back plate and a front plate including an inlet for gas to be humidified and an outlet for humidified gas.

US 4 225 542 A discloses a sterilizable evaporative humidifier for respiratory usage having a disposable absorbent evaporative element is disclosed. The humidifier provides humidification, low pressure drop and a very short warm-up period, Furthermore, electronic controls are provided to ensure safe automatic operation.

US 4 110 419 A discloses a cartridge-type humidifier apparatus embodying a separate heater module with a cylindrical opening for replaceably receiving therein disposable cylindrical humidifier cartridge modules. The cartridge modules each have a tubular metal main body adapted for a sliding fit within a complementary tubular walled heater. The metal tubular body has rigid plastic top and bottom end portions. The plastic top end portion is a cap with a center axial gas inlet tube, and a separate transverse gas delivery pipe the cap forming a closed air space over a pool of humidifying liquid. Each cartridge includes an absorption column preferably of the cylindrical tube form which is adapted to lay closely adjacent and draw water up along the cartridge's cylindrical metal body wall which serves the evaporating surface when heated by the heater modules.

US 4 657 713 A discloses a humidifier assembly. Parts of the humidifier assembly may be disposable. The assembly comprises a housing, heater means on the housing, a liquid water supply means that communicates with the heater means, a hollow shell on the housing that defines a humidification chamber, and a filter means in the humidification chamber and positioned in juxtaposition with the surface of the heater means for passing water vapour into breathable gas.

US 4921 642 A discloses a humidifier module for use in a gas humidification unit which has a humidification chamber and a water heating chamber separated by a water vapor permeable, liquid water impermeable membrane. Gas inlet and outlet ports are provided in the humidification chamber, a water inlet port is provided for the water heating chamber, a water inlet port is provided for the water heating chamber, and a heat exchange wall is provided on one side of the water heating chamber to receive heat from a heat source in the humidification assembly. The gas humidification assembly has an enclosure adjacent the heat source, for receiving the humidifier module, a water supply source, and a controlled of the heater and water supply to the humidifier module.

US 3 954 920 A discloses gas humidification system including a substantially closed chamber formed by a plurality of side walls, a bottom wall and a top wall having a gas inlet and outlet ports associated therewith, heat means associated with the bottom wall, and a humidification element removably positionable within the chamber comprising a heat conductive metallic member having an end for contact with the heat means and extending upwardly therefrom, and a layer of water absorbent material fixedly secured to at least one of the surfaces of the metallic member, the metallic member presenting a multi-faceted surface and the absorbent material paralleling the multi-faceted surface of the body portion of the metallic member thereby resenting an increase surface area for gas to pass over and around the humidification element at elevated temperatures thereby to increase the relative humidity of the gas prior to inhalation by a patient

DE 28 10 325 discloses a breathing mask with a heating system so that a patient can breathe warm air, which also includes a humidity control system, Through the middle of the cylindrical extension attached to the face piece is a tubular heating element and around this is a porous filter acting as a liquid reservoir. This can also be used as a source of treatment fluid. Between this and the face piece is mounted a hygrometer with a dial with a measuring scale over which travels a pointer so that it can be read from outside the mask. The hygrometer is mounted in an intermediate sleeve.

### SUMMARY OF INVENTION

The present invention is defined by the subject-matter of claim 1.

### BRIEF DESCRIPTION OF SEVERAL VIEWS OF THE DRAWINGS

Figure 1 shows a first embodiment of a gas warmer and/or humidifier apparatus according to the present invention;
Figure 2 shows a second embodiment of a gas warmer and/or humidifier apparatus according to the present invention having a plurality of baffles in the shell ;
Figure 3 shows a third embodiment of a gas warmer and/or humidifier apparatus according to the present invention having an external temperature or humidity sensor;
Figure 4 shows a cross section perspective view of a gas warmer and/or humidifier apparatus;
Figure 5 shows a perspective exploded view of a fourth embodiment of gas humidification apparatus according to the present invention;
Figure 6 shows a right top side perspective view of the gas humidification apparatus of Figure 5;
Figure 7 shows a right bottom side perspective view of the gas humidification apparatus of Figure 5;
Figure 8 shows a top view of the gas humidification apparatus of Figure 5;
Figure 9 shows a right side view of the gas humidification apparatus of Figure 5;
Figure 10 shows a front view of the gas humidification apparatus of Figure 5;
Figure 11 shows a rear side perspective view of the gas humidification apparatus of Figure 5;
Figure 12 shows a top view of an embodiment of a humidification material to be used with the gas humidification apparatus of Figure 5;
Figure 13 shows a perspective view of a fifth embodiment of gas humidification apparatus according to the present invention;
Figure 14 shows a side view of the gas humidification apparatus of Figure 13;
Figure 15 shows a partially exposed side view of the gas humidification apparatus of Figure 13;
Figure 16 shows a right front side and partially exposed perspective view of the gas humidification apparatus of Figure 13;
Figure 17 shows a right rear side and partially exposed perspective view of the gas humidification apparatus of Figure 13; and
Figure 18 shows a circuit diagram of heating circuit that can be used with the gas humidification apparatus/gas warmer and/or heater apparati of Figures 1-17.

### DETAILED DESCRIPTION OF THE PREFERRED EMBODIMENTS

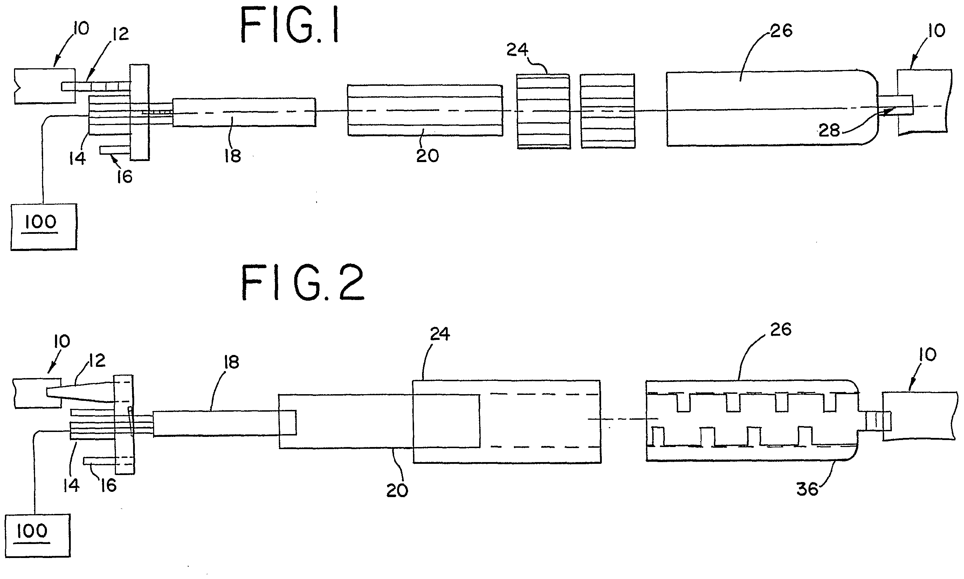

Figure 1 shows one embodiment of the gas warmer and humidification apparatus. Figure 1 shows the apparatus used in conjunction with an insufflation device. Figs. 1-3 show the apparatus 1 associated with the insufflation tubing 10. In a preferred embodiment, the apparatus is located downstream from the gas source for the insufflation device where downstream refers to a location closer to output of the insufflation tubing 10 or a patient. The apparatus 1 has an upstream end located nearer to the gas source and a downstream end located closer to the patient. The gas warmer and humidifier apparatus 1 may be constructed as a re-useable or disposable product.

As shown in Fig. 1, in one embodiment, a gas inlet port 12 is located at an upstream end of the apparatus 1 and associable with the insufflation tubing 10. A plurality of plugs 14 may also be located at the upstream end of the apparatus 1. The plugs 14 may be male leads for association with the heater 18 and/or a thermocouple and/or other suitable sensing devices. It is to be understood that the location at the upstream end is variable and other locations consistent with the characteristics of the plugs 14 are envisioned.

As shown in Figs. 1-3, the general arrangement of one embodiment of the apparatus 1 follows. The apparatus 1 includes a heater 18. Surrounding the heater 18 is a core 20. The core 20 maintains the heater 18 in a significantly watertight environment. About the core 20 is a humidification material 24. The humidification material 24 generally envelops the entire core 20. The humidification material 24 may only partially envelop the core 20 as well. A shell 26, acting as a housing, surrounds humidification material 24. At the downstream end of the apparatus 1 may be a gas outlet 28 associable with a downstream portion of the insufflation tube 10.

The heater 18 of the above embodiment may include a conventional cartridge heater, a heat generating wire, a light bulb, or other heat generating device capable of creating an elevated temperature that can radiate from the surface of the heater. As shown in Figure 1, the heater 18 is insertable within a core 20 of non-conductive material. In further embodiments, as shown in Fig. 2, the heater 18 and plugs 14 are molded into a single assembly that is then molded with the core 20 to make a single unit.

The heater 18 can be a metal structure with integral sensing elements or external sensing elements. It can also be molded of a high temperature resistant plastic. Either the metal or the plastic heater 18 is disposable, although the lower cost of the plastic heater 18 may better suit it as a disposable heater 18. Further, the disposability or re-usability of the apparatus 1 aids in maintaining the apparatus 1 sterile for any purposes that may require a sterile apparatus 1.

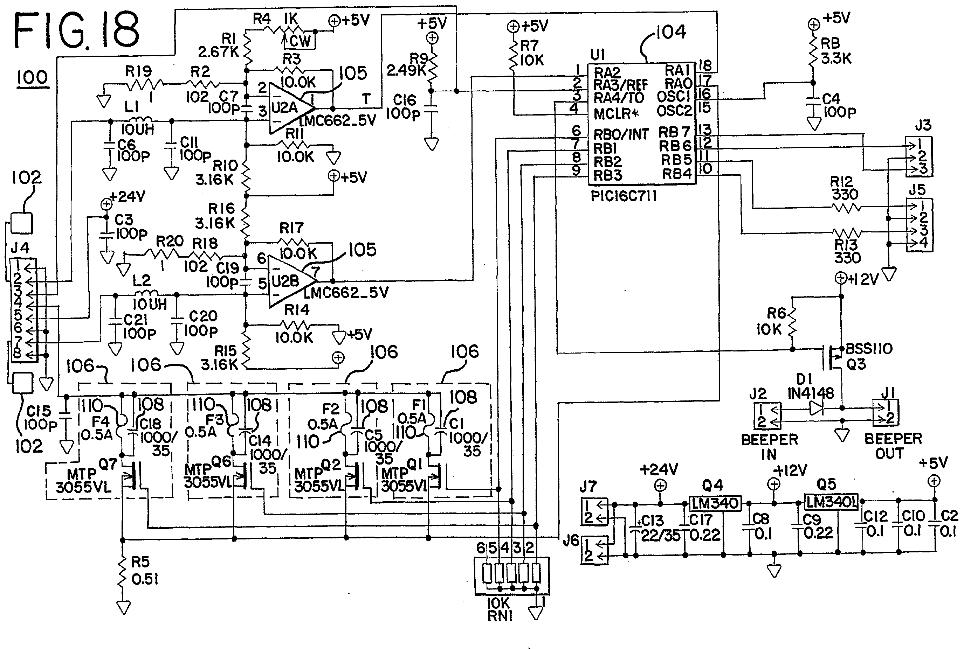

In a preferred embodiment, the heater 18 has approximately 36 watts of power although heaters 18 with other wattage, such as between 10 watts and 50 watts, can also be used. The heater 18 typically is approximately 1 to 5 inches long, preferably approximately 1½ to 3 inches long, but other sizes can be used depending on the physical size of the other components, and the amount of humidity to be generated. As shown in FIGS. 1-4 and 18, the heater 18 may be connected to control circuitry 100 controls the amount of heat and rate of heat generated by the heater 18. As shown in FIG. 18, the control circuitry 100 includes one or more temperature sensors 102 and a control system 104 to regulate the degree of energy supplied to the heater 18 by modulating the current supplied to the heater via turning on/off the current and raising or lowering the current. In the case of using two temperature sensors 102, the temperature sensors 102 each independently measure the temperature of the core 20. The temperature signals from temperature sensors 102 are continuously fed to amplifiers 105. The two signals are compared with each other and if it is determined that the difference between the signals reaches or exceeds a predetermined level, such as 5°C, then the control system 104 turns off the current drivers 106 and the current supplied to the heater 18. The current drivers 106 are turned off because reaching or exceeding the predetermined level denotes that one or both of the sensors 102 are defective and need to be replaced.

Assuming that the sensors 102 are not deemed defective, the control system 104 includes four identical current drivers 106 that are in parallel with one another as shown in FIG. 18. Each driver 106 provides an output that is identical with the outputs of the other three drivers 106. The control system 104 will drive each of the outputs of the current drivers 106 with approximately a 25% duty cycle wave shape. The four drivers combined will provide approximately 100% drive to the heater 18. Each driver 106 includes a capacitor 108 of 1000 *µ*F in parallel with a fuse 110. The capacitors 108 direct the current during its respective 25% duty cycle away from its corresponding fuse 110. In the event that a single driver 106 fails, allowing continuous current flow, the corresponding capacitor 108 will charge up and allow current to flow through the corresponding fuse 110. In less than approximately 2 seconds, the fuse 110 in the driver circuit 106 will create an open circuit, thus preventing uncontrolled current to flow to the heater 18.

In one embodiment, the apparatus 1 can have wiring to the heater 18 permanently attached. In another embodiment shown in Fig. 1, the apparatus 1 can have wiring to the heater 18 constructed with an integral connector that can be molded into the apparatus 1 or connected/disconnected via a one time use tab connection system. In yet another embodiment, the apparatus 1 can have wiring to the heater 18 with the terminations molded into a natural connector, so that the cabling can be plugged into it, reducing its cost. The electronic wiring used to provide power and to measure the temperature or humidity can be wired directly to the active elements and over molded. In the preferred embodiment, the output wires will be molded or inserted into the shell 26 in order to make the cord detachable from the apparatus 1.

The heater 18 may be controlled by conventional heater controllers as are available on the market, such as those made by Watlow. Controllers typically are designed to work with temperature sensing devices such as thermocouples resistance temperature detectors (RTD's) and or thermistors.

Optionally, in further embodiments, the apparatus 1 can be provided with additional circuitry to measure humidity using a humidity sensor. Humidity sensors are available through Omega Engineering located in Atlanta, Georgia, which can supply both the sensor and circuitry for reading and display. Additionally, optionally, the temperature of the gas and the humidity of the gas could be displayed with additional circuitry. A remote power unit, part of the insufflator, or part of any other device used in the Operating Room associated with endoscopic procedures could provide the additional circuitry to display this information. Based on the readings, adjustments could be made on the amount of moisture fed to the humidification material 24, or how much heat should be applied, or both.

In one embodiment, control could also be tied to the insufflator to supply the circuitry mentioned above. By monitoring characteristics in temperature, gas volume used, gas flow rate and/or humidity readings, the insufflator could dynamically control the variables to maintain optimum conditions.

The core 20 may be made of, but not limited to, plastic or a sheet metal. Some of the plastics that may be used for the core 20 include polycarbonate, Ryton™, Vespel™, or any of the high temperature plastics. A sheet metal such as aluminum coated with a non-conductive substance may also be used for the core 20.

As shown in Figure 1, the apparatus 1 includes a humidification material 24. The humidification material 24 both readily absorbs moisture and readily releases it when exposed to a dry environment. Materials such as nylon and cotton are just a few of the many commercially available fibers that can meet these requirements. The humidification material 24 can have a tubular inside and outside surface. Tubular refers to a smooth surface. Yet, it is envisioned in further embodiments that the humidification material 24 may have a patterned or varying 15 degrees of a non-smooth surface.

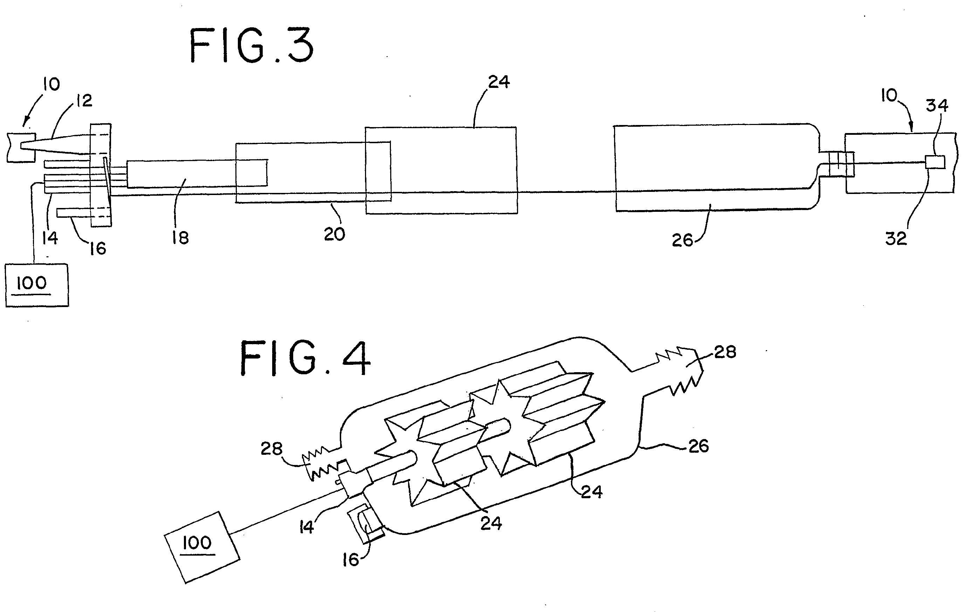

As shown in Fig. 4, the humidification material 24 used in the preferred embodiment has a smooth inner surface and a serrated or star-like shaped outer surface to maximize surface area in the shortest possible linear space. Fig. 4 shows the preferred embodiment including a first and a second section of the humidification material 24. Each section of the humidification material 24 is approximately an inch long with an inner channel in intimate contact with the heater 18. Each of these serrated sections is slid over the core 20 that contains the heater 18. Preferably, a ¼ inch gap should be between the serrated sections. In one embodiment, a plastic spacer may be inserted between the serrated sections to provide the gap. In a preferred embodiment, the first and second serrated sections should be set out of phase with each other to force turbulence of the gas and increase the surface area of the material as it passes over the sections. Note that the first and second serrated sections can be formed from a single serrated material by cutting the single serrated material so that the two serrated sections are formed. After cutting, the two serrated sections are rotated relative to one another until the desired phase difference between the two sections is achieved.

The flow of CO₂ gas over the absorbent material is affected by the shape of the absorbent material and/or the channel within the shell 26. In one embodiment, the absorbent humidification material 24 may be cylindrically shaped and surrounded by a coil used to direct the flow of CO₂ gas. As the CO₂ gas travels through the windings of the coil, warmth and humidity are transferred to the CO₂ gas. The external surfaces of the coil rest against the inside of the shell 26 forming a seal that forces the CO₂ gas to travel through or within the coil windings.

Other shapes and sizes can be used for the humidification material 24. Manufacturers of this humidification material 24 are Pall Medical located in East Hills, New York and Filtrona Richmond Inc. located in Richmond, Virginia.

The encased heater 18 elevates the temperature of the humidification material 24 thereby elevating the temperature of the moisture it contains. The elevated temperature of the moisture leads to the creation of a vapor absorbed into the gas as it flows over the humidification material 24. Preferably, the humidification material 24 has a configuration that presents a high surface area to the direction of gas flow to allow increased opportunity for the moisture to evaporate into the gas thereby humidifying the gas.

In a further embodiment, shown for example in Fig. 2, turbulence of the gas is created by the interior of the shell 26 covering the humidification material 24 and heater 18 having a surface area that is of an irregular pattern or texture. This turbulence may be created using a variety of structures. These structures may be located, for example, on or as part of the shell 26 or humidification material 24. Further example of a structure for creating turbulence may be a spiral barrier. In additional embodiments, other structures may be incorporated, for example, by being either attached to the humidification material 24 or interior of the shell 26 of the apparatus 1.

The moisture applied to the humidification material 24 can contain medications or additives that will evaporate and be carried along in the humidified gas to the patient. Levels of medication and/or fluid in the gas can be controlled by timed evaporation and adsorption rates. Fluid could be infused by syringe, gravity feed through tubing, or by any number of pumps, to retain proper saturation levels.

The apparatus 1 will have a port 16 for the infusion of fluid for the production of moisture. Moisture may include sterile water, medication, or a mixture of fluids required for merely humidification or dispensing of medication. The port 16 can be of the standard injection port used typically in the medical industry, a valve, or any other device, which can open or close allowing for the entrance of the fluid.

The apparatus 1 includes one or more temperature sensing devices (not shown) to regulate the heater 18. Each temperature-sensing device can be a resistive temperature device (RTD), a thermister, or a thermocouple. In the preferred embodiment, a K type thermocouple is embedded inside the heater 18 to measure its temperature. Any number of heater controller manufacturers such as Watlow or Hot Watt can provide the temperature sensing and control device. As shown in Figure 1, the shell 26 is an oblong tube having an internal channel, but any shape that will accommodate the internal elements of the device is acceptable. In the preferred embodiment, the internal channel of the shell 26 will be smooth. In a further embodiment, any form of surface irregularity to promote turbulence without flow restriction is acceptable for the 15 internal channel of the shell 26. The shell 26 has an output opening 28 and an input opening 12 for the gas. The shell 26 additionally has a fluid fill port 16 for the infusion of fluid. Although, other methods of inserting the appropriate fluid or medicine in the shell 26 are possible.

Overall length of the preferred embodiment will be between 3½ and 4 inches. Preferably, the apparatus 1 will weigh approximately four ounces. The shell 26 can be made of any suitable material, for example, metal or plastic.

In additional embodiments, as shown in Figure 3, a humidity sensor 34 may be included in the apparatus 1. Appropriate humidity sensors 34 can be obtained from Omega Corporation located in Atlanta, Georgia.

Optionally, in further embodiments, in addition to the temperature sensing device described above, an external temperature sensing device 32 can be inserted in the insufflation tubing 10 just outside of the gas outlet 28. The same types of temperature sensing devices internal to the apparatus 1 as described above can be used. This device 32 measures the downstream temperature of the gas.

The temperature of the gas is related to the temperature of the heater 18. The temperature sensing device located within the heater 18 measures the temperature of the heater 18. The temperature of the gas is not directly measured. Rather, the resulting temperature of the gas correlates to the temperature of the heater.

The warmed and humidified gas leaves the apparatus 1 through a gas outlet 28. The gas outlet may be a series of holes. The gas then enters the insufflation tubing 10 for possible delivery to a patient.

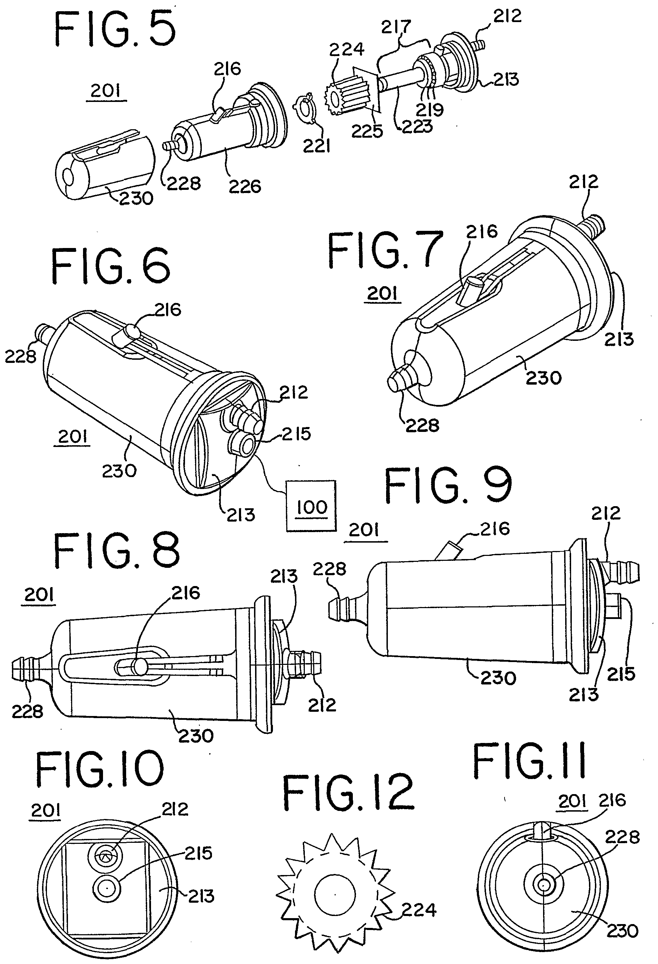

Another embodiment of a gas humidification apparatus is shown in Figs. 5-12. In a manner similar to the devices of Figs. 1-4, the gas humidification apparatus 201 can be used in conjunction with an insufflation device. In particular the gas humidification apparatus 201 is located downstream from a gas source for the insufflation device. The gas humidification apparatus 201 may be constructed as a re-useable or disposable product.

As shown in Figs. 5, 6, 9 and 10, a gas inlet port 212 is attached through a side portion of a front cap 213 of the gas humidification apparatus 201. In addition, an inlet port 215 is attached through a central portion of the front cap 213. The inlet port 215 allows for electrical components and wiring to be inserted into the gas humidification apparatus 201. The gas humidification apparatus 201 can be modified so that the ports 212 and 215 are interchanged with one another.

As shown in Fig. 5, the cap 213 includes an annular metallic heater housing 217 that is attached thereto. The heater housing 217 is in fluid communication with the gas inlet port 212. The heater housing 217 contains a heater cartridge that is well known in the art. When activated the heater cartridge heats up the interior and body of the heater housing 217 so that gases within and outside the heater housing 217 are heated. The heater housing 217 also includes a plurality of circular holes 219 having a diameter of approximately 0.1" (0.254cm). Other shapes and sizes for the holes 219 are possible, such as triangular and square shaped openings. When gas flows into the gas humidification apparatus 201 via the gas inlet port 212, the gas flows into the heater housing 217, where it is heated if necessary, and then flows out of the holes 219. As shown in Fig. 5, there are approximately sixteen holes 219 that are arranged equidistantly from one another along an annular ring. The holes 219 of the heater housing 217 improve the rate of heating of the gas within the gas humidification apparatus 201 and create turbulence for the gas flowing within the gas humidification apparatus 201.

Two of the holes 219 preferably have their own RTD sensor. These sensors operate in the same manner as the temperature sensors for the embodiments of Figs. 1-4. In particular, the temperature measured by the two sensors are compared with one another to determine if one or both of the sensors is defective.

As shown in Fig. 5, a rear cylindrical portion 223 of the heater housing 217 is snugly inserted into a cylindrical central opening of a humidification material 224 that is preferably made of the same material as the humidification materials 24 described previously with respect to Figs. 1-4. A washer 221 is fitted over the rear portion 223 and abuts against the rear face of the humidification material 224 and acts as a stop in that it prevents the humidification material 224 from slipping off of the rear portion 223 and being wedged into an outlet 228.

In an alternative embodiment, the gas humidification apparatus 201 can further include a plate 225 positioned between the front or proximal end of the humidification material 224 and the heater housing 217. Since the holes 219 face the front end of the humidification material 224, the plate 225 allows the gas to flow along the exposed side of the humidification material 224. Note that the gas will flow along the side of the humidification material 224 with or without the presence of the plate 225.

As shown in Fig. 12, the humidification material 224 has a star-like pattern with ten to twelve points that aid in generating turbulence in the gas within the gas humidification apparatus 201 in a similar manner that the humidification material 24 of Figs. 1 and 4 do.

In an alternative embodiment, a second humidification material 224 may be spaced from the first humidification material by a spacer and out of phase with the first humidification material in the same manner as described previously with respect to the embodiment of Figs. 1 and 4.

As shown in FIG. 5, the assembled humidification material 224 and washer 221 and the inlet port 215 and the heater housing 217 are inserted into a housing or shell 226. After insertion, the front cap 213 is screwed on or snap fit onto the heater housing 217. The housing 226 is made of a suitable material, such as plastic or metal, and has a downstream outlet 228 that allows the gas to flow outside of the housing 226.

As shown in FIGS. 5-9 and 11, the housing 226 includes a port 216 that allows fluid to be infused by syringe, gravity feed through tubing, or by any number of pumps, to the humidification material 224. The fluids infused may include sterile water, medication, or a mixture of fluids required for merely humidification or dispensing of medication. The interior end of the port 216 is positioned so that infused fluids drip into the housing 226 and are soaked up by the entire humidification material 224 by capillary action. The port 216 is similar to the port 16 described previously with respect to the embodiments of FIGS. 1-4.

As shown in FIGS. 5-9, the housing 226 is inserted into a sleeve or shroud 230 so that the port 216 is slid along a slit 232 formed in the sleeve 230 and the outlet 228 extends through a rear opening 234 of the sleeve 230. The sleeve 230 is snap fit to the housing 226. The sleeve 230 is made of a thermal insulation material that retains the heat within the housing 226 so that a person can handle the sleeve 230 without fear of being exposed to excessive heat and without significantly heating up the ambient atmosphere.

Note that the sleeve 230, the housing 226 and the humidification material 224 may be disposable while the cap 213 and its attached heater housing 217 may be reusable.

The gas humidification apparatus 201 may include the temperature sensors, humidity sensors and control circuitry previously described with respect to the embodiments of FIGS. 1-4 and 18 so that the temperature and humidity of the gas flowing within the apparatus and delivered to a patient via outlet 228 is controlled.

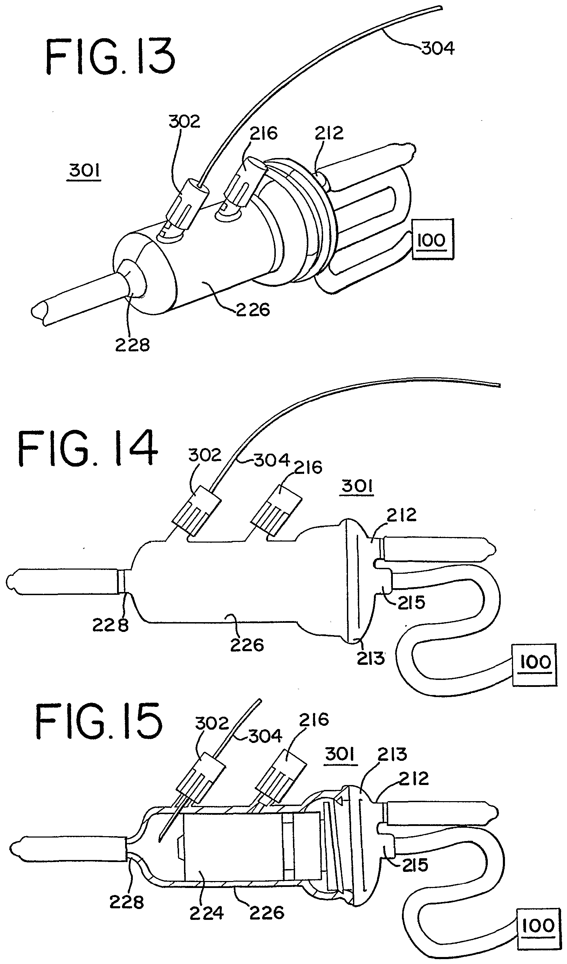

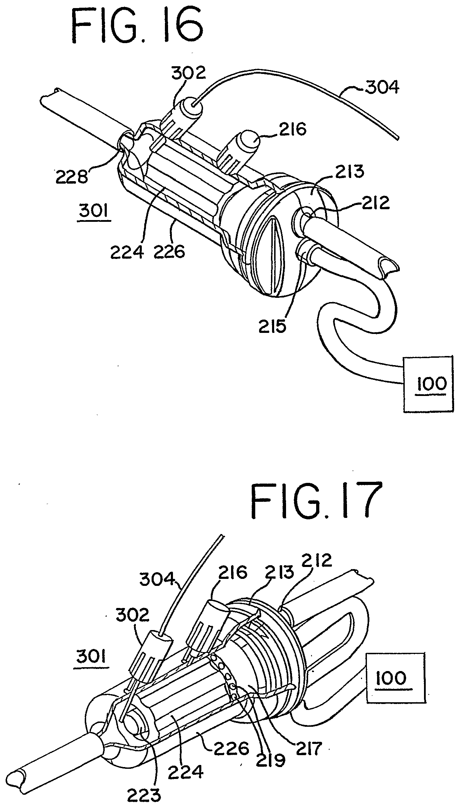

Another embodiment of a gas humidification apparatus is shown in Figs. 13-17. The gas humidification apparatus 301 essentially has the same structure as the gas humidification apparatus 201 of Figs. 5-12 and so like components will be designated with like numerals. One difference is that a second port 302 is added to the housing 226. The second port 302 is positioned between the humidification material 224 and the outlet 228 so as to allow a distal end of a catheter 304 to be inserted into the port 302. Depending on the intended material to be delivered to the patient, the distal end of the catheter 304 may be positioned within the port 302, within the interior of the gas humidification apparatus 301 or within a tube attached to the outlet 228 and in fluid communication with a section of a patient, or within the section of the patient. An example of a catheter that can be inserted into the gas humidification apparatus 201 is the catheter described in U.S. Patent No. 5,964,223, the entire contents of which are incorporated herein by reference. Other devices can be inserted into the port 302 in a similar manner as described above with respect to catheter 304, such as a lumen and an endoscope. Furthermore, gases, liquids, aerosols and medicines may be conveyed to a patient by a tube or other know dispensing devices inserted through the port 302 and exiting out of the outlet 228 into the patient. Note that the materials dispensed into the port 302 by the above-mentioned dispensing devices may have properties that raise the humidity of the gas within the interior of the gas humidification apparatus 301.

The gas humidification apparatus 301 may include the temperature sensors, humidity sensors and control circuitry previously described with respect to the embodiments of FIGS. 1-4 and 18 so that the temperature and humidity of the gas flowing within the apparatus and delivered to a patient is controlled.

In each of the devices for humidifying and/or warming a gas described previously with respect to FIGS. 1-18, it is desired that the flowing gas achieves a humidity that ranges from approximately 80 to 100 percent humidity and achieves a temperature that ranges from approximately 90 to 105 degrees Fahrenheit at a constant flow rate of approximately 20 liters per minute.

The embodiments of the invention disclosed herein are presently considered to be preferred, various changes and modifications can be made without departing from the subject-matter claimed. As noted, the discussion above is descriptive, illustrative and exemplary and is not to be taken as limiting the scope defined by any appended claims, and all changes that come within the meaning and range of equivalents are intended to be embraced therein.

## Claims

1. A gas humidification apparatus (1) comprising:
inlet means (12) for supplying a gas,
a humidification device in fluid communication with said inlet means (12), said humidification device comprising a humidification material (24) that readily absorbs moisture and readily releases moisture when exposed to a dry environment,
outlet means (28) in fluid communication with said humidification device for expelling said gas from said gas humidification apparatus (1), wherein said gas humidification apparatus comprises turbulence means for generating turbulence in said gas, and in that said humidification apparatus (1) further comprises a heater (18), wherein said humidification device envelops said heater (18),
wherein said humidification material (24) has a configuration that generates turbulence in said gas as it passes over a surface of said humidification material (24), **characterized in that** said surface of said humidification material (24) is serrated or star-shaped.

2. The gas humidification apparatus of claim 1, wherein said surface of said humidification material (24) varies 15 degrees of a non-smooth surface.

3. The gas humidification apparatus of claim 1, wherein said surface of said humidification material (24) is irregularly patterned.

4. The gas humidification apparatus of claim 1, wherein said surface of said humidification material (24) is maximized in surface area in the shortest possible linear space.

5. The gas humidification apparatus of claim 1, wherein said humidification device further comprises a second humidification material that is spaced from said humidification material (24) and readily absorbs moisture and readily releases moisture when exposed to a dry environment, wherein said second humidification material has a configuration that generates turbulence for a gas that should pass over a surface of said second humidification surface.

6. The gas humidification apparatus of claim 5, further comprising a spacer interposed between said humidification material (24) and said second humidification material.

7. The gas humidification apparatus of claim 5, wherein said surface of said second humidification material is patterned.

8. The gas humidification apparatus of claim 7, wherein said surface of said second humidification material is out of phase with respect to said surface of said humidification material (24).

9. The gas humidification apparatus of claim 1, wherein said humidification material (24) comprises a medication infused therein.

10. The gas humidification apparatus of one of claims 1 to 9, wherein said heater (18) is positioned separate from said humidification device.

11. The gas humidification apparatus of claim 10, wherein said heater (18) is positioned upstream from said humidification device.

12. The gas humidification apparatus of one of claims 1 to 11, further comprising a core (20) into which said heater is inserted.

13. The gas humidification apparatus of claim 12, wherein said core (20) comprises a non-conductive material.

14. The gas humidification apparatus of claim 1, further comprising a humidity sensor (34) for measuring humidity of a gas that flows within said gas humidification apparatus.

15. The gas humidification apparatus of claim 1, further comprising a temperature sensor (102) for measuring a temperature of a gas that flows within said gas humidification apparatus.

16. The gas humidification apparatus of claim 14, further comprising a temperature sensor (102) for measuring a temperature of said gas that flows within said gas humidification apparatus.

17. The gas humidification apparatus of claim 15, wherein said temperature sensor (102) measures said temperature of said gas in an indirect manner.

18. The gas humidification apparatus of claim 1, further comprising a temperature sensor (102) for measuring a temperature of a gas that flows within said gas humidification apparatus.

19. The gas humidification apparatus of claim 18, wherein said temperature sensor (102) measures said temperature of said gas in an indirect manner by measuring a temperature of said heater (18).

20. The gas humidification apparatus of claim 14, further comprising a control device connected to said humidity sensor (34) that controls humidity within said gas humidification apparatus based on a signal from said humidity sensor (34).

21. The gas humidification apparatus of claim 15, further comprising a control device (100) connected to said temperature sensor (102) that controls temperature within said gas humidification apparatus based on a signal from said temperature sensor (102).

22. The gas humidification apparatus of claim 16, further comprising a control device connected to said humidity sensor (34) and said temperature sensor (102) that controls humidity and temperature within said gas humidification apparatus based on signals from said humidity sensor (34) and said temperature sensor (32).

23. The gas humidification apparatus of claim 2, further comprising: a port (16) in fluid communication with said gas humidification apparatus; and a catheter inserted into said port (16).

24. The gas humidification apparatus of claim 1,
wherein said humidification material (24) is placed within a shell (26) that has a configuration that generates turbulence in said gas as it passes over a surface of said shell (26).

25. The gas humidification apparatus of claim 24, further comprising a coil positioned adjacent to said shell (26) that forces a gas to travel through or within said coil.

26. The gas humidification apparatus of claim 24, wherein said shell (26) comprises an interior surface with an irregular pattern.

27. The gas humidification apparatus of claim 24, wherein said shell (26) comprises an interior surface with a spiral barrier.

28. The gas humidification apparatus of claim 24, wherein said humidification material (24) is cylindrical in shape.

29. The gas humidification apparatus of claim 24, wherein said humidification material (24) comprises a medication infused therein.

30. The gas humidification apparatus of claim 1, wherein said heater housing (217) comprises a plurality of openings (219), said plurality of openings (219) having a configuration that generates turbulence in said gas as it passes through said plurality of openings (219).

31. The gas humidification apparatus of claim 25, wherein said humidification device further comprises a second humidification material that is spaced from said humidification material (24) and readily absorbs moisture and readily releases moisture when exposed to a dry environment, wherein said second humidification material has a configuration that generates turbulence for a gas that should pass over a surface of said second humidification surface.

32. The gas humidification apparatus of claim 31, further comprising a spacer interposed between said humidification material (24) and said second humidification material.

33. The gas humidification apparatus of claim 31, wherein said surface of said second humidification material is patterned.

34. The gas humidification apparatus of claim 33, wherein said surface of said second humidification material is out of phase with respect to said surface of said humidification material (24).

35. The gas humidification apparatus of claim 25, wherein said humidification material (24) comprises a medication infused therein.

36. The gas humidification apparatus of claim 25, further comprising a humidity sensor (34) for measuring humidity of a gas that flows within said gas humidification apparatus.

37. The gas humidification apparatus of claim 25, further comprising a temperature sensor (102) for measuring a temperature of a gas that flows within said gas humidification apparatus.

38. The gas humidification apparatus of claim 36, further comprising a temperature sensor (102) for measuring a temperature of said gas that flows within said gas humidification apparatus.

39. The gas humidification apparatus of claim 37, wherein said temperature sensor (102) measures said temperature of said gas in an indirect manner.

40. The gas humidification apparatus of claim 36, further comprising a control device connected to said humidity sensor (34) that controls humidity within said gas humidification apparatus based on a signal from said humidity sensor (34).

41. The gas humidification apparatus of claim 37, further comprising a control device (100) connected to said temperature sensor (102) that controls temperature within said gas humidification apparatus based on a signal from said temperature sensor (102).

42. The gas humidification apparatus of claim 38, further comprising a control device (100) connected to said humidity sensor (34) and said temperature sensor (102) that controls humidity and temperature within said gas humidification apparatus based on signals from said humidity sensor (34) and said temperature sensor (102).

43. The gas humidification apparatus of claim 25, further comprising: a port (16) in fluid communication with said gas humidification apparatus; and a catheter inserted into said port (16).

44. The gas humidification apparatus of claim 1, further comprising: a port (16) in fluid communication with said gas humidification apparatus; and a catheter inserted into said port (16).

## Patentansprüche

1. Gas-Befeuchtungsvorrichtung (1), die umfasst:
eine Einlasseinrichtung (12) zum Zuführen eines Gases,
eine Befeuchtungseinrichtung, die in Fluidverbindung mit der Einlasseinrichtung (12) steht, wobei die Befeuchtungseinrichtung ein Befeuchtungsmaterial (24) umfasst, das Feuchtigkeit leicht absorbiert und Feuchtigkeit leicht freisetzt, wenn es einer trockenen Umgebung ausgesetzt ist,
eine Auslasseinrichtung (28), die in Fluidverbindung mit der Befeuchtungseinrichtung steht, um das Gas aus der Gas-Befeuchtungsvorrichtung (1) auszustoßen,
wobei
die Gas-Befeuchtungsvorrichtung eine Turbulenz-Einrichtung zum Erzeugen von Turbulenz in dem Gas umfasst, und
die Befeuchtungsvorrichtung (1) des Weiteren eine Heizeinrichtung (18) umfasst, wobei die Befeuchtungseinrichtung die Heizeinrichtung (18) umschließt,
und das Befeuchtungsmaterial (24) eine Form hat, durch die Turbulenz in dem Gas erzeugt wird, wenn es über eine Oberfläche des Befeuchtungsmaterials (24) strömt,
**dadurch gekennzeichnet, dass**
die Oberfläche des Befeuchtungsmaterials (24) gezackt oder sternförmig ist.

2. Gas-Befeuchtungsvorrichtung nach Anspruch 1, wobei die Oberfläche des Befeuchtungsmaterials (24) um 15 Grad einer nicht-glatten Oberfläche variiert.

3. Gas-Befeuchtungsvorrichtung nach Anspruch 1, wobei die Oberfläche des Befeuchtungsmaterials (24) unregelmäßig strukturiert ist.

4. Gas-Befeuchtungsvorrichtung nach Anspruch 1, wobei die Oberfläche des Befeuchtungsmaterials (24) in dem kürzestmöglichen linearen Raum maximale Oberfläche hat.

5. Gas-Befeuchtungsvorrichtung nach Anspruch 1, wobei die Befeuchtungseinrichtung des Weiteren ein zweites Befeuchtungsmaterial umfasst, das von dem Befeuchtungsmaterial (24) beabstandet ist und Feuchtigkeit leicht absorbiert und Feuchtigkeit leicht freisetzt, wenn es einer trockenen Umgebung ausgesetzt ist, und das zweite Befeuchtungsmaterial eine Form hat, durch die Turbulenz für ein Gas erzeugt wird, das über eine Oberfläche des zweiten Befeuchtungsmaterials strömen sollte.

6. Gas-Befeuchtungsvorrichtung nach Anspruch 5, die des Weiteren einen Abstandshalter umfasst, der zwischen dem Befeuchtungsmaterial (24) und dem zweiten Befeuchtungsmaterial angeordnet ist.

7. Gas-Befeuchtungsvorrichtung nach Anspruch 5, wobei die Oberfläche des zweiten Befeuchtungsmaterials strukturiert ist.

8. Gas-Befeuchtungsvorrichtung nach Anspruch 7, wobei die Oberfläche des zweiten Befeuchtungsmaterials phasenversetzt in Bezug auf die Oberfläche des Befeuchtungsmaterials (24) ist.

9. Gas-Befeuchtungsvorrichtung nach Anspruch 1, wobei das Befeuchtungsmaterial (24) ein darin infundiertes Medikament umfasst.

10. Gas-Befeuchtungsvorrichtung nach einem der Ansprüche 1 bis 9, wobei die Heizeinrichtung (18) separat von der Befeuchtungseinrichtung positioniert ist.

11. Gas-Befeuchtungsvorrichtung nach Anspruch 10, wobei die Heizeinrichtung (18) stromauf von der Befeuchtungseinrichtung positioniert ist.

12. Gas-Befeuchtungsvorrichtung nach einem der Ansprüche 1 bis 11, die des Weiteren einen Kern (20) umfasst, in den die Heizeinrichtung eingeführt ist.

13. Gas-Befeuchtungsvorrichtung nach Anspruch 12, wobei der Kern (20) ein nichtleitendes Material umfasst.

14. Gas-Befeuchtungsvorrichtung nach Anspruch 1, die des Weiteren einen Feuchtigkeits-Sensor (34) zum Messen von Feuchtigkeit eines Gases umfasst, das im Inneren der Gas-Befeuchtungsvorrichtung strömt.

15. Gas-Befeuchtungsvorrichtung nach Anspruch 1, die des Weiteren einen Temperatur-Sensor (102) zum Messen einer Temperatur eines Gases umfasst, das im Inneren der Gas-Befeuchtungsvorrichtung strömt.

16. Gas-Befeuchtungsvorrichtung nach Anspruch 14, die des Weiteren einen Temperatur-Sensor (102) zum Messen einer Temperatur des Gases umfasst, das im Inneren der Gas-Befeuchtungsvorrichtung strömt.

17. Gas-Befeuchtungsvorrichtung nach Anspruch 15, wobei der Temperatur-Sensor (102) die Temperatur des Gases indirekt misst.

18. Gas-Befeuchtungsvorrichtung nach Anspruch 1, die des Weiteren einen Temperatur-Sensor (102) zum Messen einer Temperatur eines Gases umfasst, das im Inneren der Gas-Befeuchtungsvorrichtung strömt.

19. Gas-Befeuchtungsvorrichtung nach Anspruch 18, wobei der Temperatur-Sensor (102) die Temperatur des Gases indirekt misst, indem er eine Temperatur der Heizeinrichtung (18) misst.

20. Gas-Befeuchtungsvorrichtung nach Anspruch 14, die des Weiteren eine Steuereinrichtung umfasst, die mit dem Feuchtigkeits-Sensor (34) verbunden ist und Feuchtigkeit im Inneren der Gas-Befeuchtungsvorrichtung auf Basis eines Signals von dem Feuchtigkeits-Sensor (34) steuert.

21. Gas-Befeuchtungsvorrichtung nach Anspruch 15, die des Weiteren eine Steuereinrichtung (100) umfasst, die mit dem Temperatur-Sensor (102) verbunden ist und Temperatur im Inneren der Gas-Befeuchtungsvorrichtung auf Basis eines Signals von dem Temperatur-Sensor (102) steuert.

22. Gas-Befeuchtungsvorrichtung nach Anspruch 16, die des Weiteren eine Steuereinrichtung umfasst, die mit dem Feuchtigkeits-Sensor (34) und dem Temperatur-Sensor (102) verbunden ist und Feuchtigkeit sowie Temperatur im Inneren der Gas-Befeuchtungsvorrichtung auf Basis von Signalen von dem Feuchtigkeits-Sensor (34) und dem Temperatur-Sensor (32) steuert.

23. Gas-Befeuchtungsvorrichtung nach Anspruch 2, die des Weiteren umfasst:
einen Anschluss (16), der in Fluidverbindung mit der Gas-Befeuchtungsvorrichtung steht; und
einen Katheter, der in den Anschluss (16) eingeführt ist.

24. Gas-Befeuchtungsvorrichtung nach Anspruch 1,
wobei das Befeuchtungsmaterial (24) in eine Hülse (26) eingesetzt ist, die eine Form hat, die Turbulenz in dem Gas erzeugt, wenn es über eine Oberfläche der Hülse (26) strömt.

25. Gas-Befeuchtungsvorrichtung nach Anspruch 24, die des Weiteren eine Schlange umfasst, die an die Hülse (26) angrenzend positioniert ist und ein Gas zwingt, durch oder in der Schlange zu strömen.

26. Gas-Befeuchtungsvorrichtung nach Anspruch 24, wobei die Hülse (26) eine Innenfläche mit einer unregelmäßigen Struktur umfasst.

27. Gas-Befeuchtungsvorrichtung nach Anspruch 24, wobei die Hülse (26) eine Innenfläche mit einer spiralförmigen Sperre umfasst.

28. Gas-Befeuchtungsvorrichtung nach Anspruch 24, wobei das Befeuchtungsmaterial (24) zylindrisch geformt ist.

29. Gas-Befeuchtungsvorrichtung nach Anspruch 24, wobei das Befeuchtungsmaterial (24) ein darin infundiertes Medikament umfasst.

30. Gas-Befeuchtungsvorrichtung nach Anspruch 1, wobei das Gehäuse (217) der Heizeinrichtung eine Vielzahl von Öffnungen (219) umfasst und die Vielzahl von Öffnungen (219) eine Form haben, die Turbulenz in dem Gas erzeugt, wenn ist durch die Vielzahl von Öffnungen (219) strömt.

31. Gas-Befeuchtungsvorrichtung nach Anspruch 25, wobei die Befeuchtungseinrichtung des Weiteren ein zweites Befeuchtungsmaterial umfasst, das von dem Befeuchtungsmaterial (24) beabstandet ist und Feuchtigkeit leicht absorbiert und Feuchtigkeit leicht freisetzt, wenn es einer trockenen Umgebung ausgesetzt ist, und das zweite Befeuchtungsmaterial eine Form hat, durch die Turbulenz für ein Gas erzeugt wird, das über eine Oberfläche des zweiten Befeuchtungsmaterials strömen sollte.

32. Gas-Befeuchtungsvorrichtung nach Anspruch 31, die des Weiteren einen Abstandshalter umfasst, der zwischen dem Befeuchtungsmaterial (24) und dem zweiten Befeuchtungsmaterial angeordnet ist.

33. Gas-Befeuchtungsvorrichtung nach Anspruch 31, wobei die Oberfläche des zweiten Befeuchtungsmaterials strukturiert ist.

34. Gas-Befeuchtungsvorrichtung nach Anspruch 33, wobei die Oberfläche des zweiten Befeuchtungsmaterials phasenversetzt in Bezug auf die Oberfläche des Befeuchtungsmaterials (24) ist.

35. Gas-Befeuchtungsvorrichtung nach Anspruch 25, wobei das Befeuchtungsmaterial (24) ein darin infundiertes Medikament umfasst.

36. Gas-Befeuchtungsvorrichtung nach Anspruch 25, die des Weiteren einen Feuchtigkeits-Sensor (34) zum Messen von Feuchtigkeit eines Gases umfasst, das im Inneren der Gas-Befeuchtungsvorrichtung strömt.

37. Gas-Befeuchtungsvorrichtung nach Anspruch 25, die des Weiteren einen Temperatur-Sensor (102) zum Messen einer Temperatur eines Gases umfasst, das im Inneren der Gas-Befeuchtungsvorrichtung strömt.

38. Gas-Befeuchtungsvorrichtung nach Anspruch 36, die des Weiteren einen Temperatur-Sensor (102) zum Messen einer Temperatur eines Gases umfasst, das im Inneren der Gas-Befeuchtungsvorrichtung strömt.

39. Gas-Befeuchtungsvorrichtung nach Anspruch 37, wobei der Temperatur-Sensor (102) die Temperatur des Gases indirekt misst.

40. Gas-Befeuchtungsvorrichtung nach Anspruch 36, die des Weiteren eine Steuereinrichtung umfasst, die mit dem Feuchtigkeits-Sensor (34) verbunden ist und Feuchtigkeit im Inneren der Gas-Befeuchtungsvorrichtung auf Basis eines Signals von dem Feuchtigkeits-Sensor (34) steuert.

41. Gas-Befeuchtungsvorrichtung nach Anspruch 37, die des Weiteren eine Steuereinrichtung (100) umfasst, die mit dem Temperatur-Sensor (102) verbunden ist und Temperatur im Inneren der Gas-Befeuchtungsvorrichtung auf Basis eines Signals von dem Temperatur-Sensor (102) steuert.

42. Gas-Befeuchtungsvorrichtung nach Anspruch 38, die des Weiteren eine Steuereinrichtung (100) umfasst, die mit dem Feuchtigkeits-Sensor (34) und dem Temperatur-Sensor (102) verbunden ist und Feuchtigkeit sowie Temperatur im Inneren der Gas-Befeuchtungsvorrichtung auf Basis von Signalen von dem Feuchtigkeits-Sensor (34) und dem Temperatur-Sensor (102) steuert.

43. Gas-Befeuchtungsvorrichtung nach Anspruch 25, die des Weiteren umfasst:
einen Anschluss (16), der in Fluidverbindung mit der Gas-Befeuchtungsvorrichtung steht; und
einen Katheter, der in den Anschluss (16) eingeführt ist.

44. Gas-Befeuchtungsvorrichtung nach Anspruch 1, die des Weiteren umfasst:
einen Anschluss (16), der in Fluidverbindung mit der Gas-Befeuchtungsvorrichtung steht; und
einen Katheter, der in den Anschluss (16) eingeführt ist.

## Revendications

1. Appareil d'humidification de gaz (1) comprenant :
un moyen orifice d'entrée (12) pour l'alimentation d'un gaz,
un dispositif d'humidification en communication fluide avec ledit moyen orifice d'entrée (12), ledit dispositif d'humidification comprenant un matériau d'humidification (24) qui absorbe facilement l'humidité et libère facilement l'humidité lorsqu'il est exposé à un environnement sec,
un moyen orifice de sortie (28) en communication fluide avec ledit dispositif d'humidification pour expulser ledit gaz dudit appareil d'humidification de gaz (1),
où
ledit appareil d'humidification de gaz comprend un moyen de turbulence pour générer une turbulence dans ledit gaz, et en ce que
ledit appareil d'humidification (1) comprend en outre un dispositif de chauffage (18), où ledit dispositif d'humidification enveloppe ledit dispositif de chauffage (18),
où ledit matériau d'humidification (24) présente une configuration qui génère une turbulence dans ledit gaz lorsqu'il passe sur une surface dudit matériau d'humidification (24),
**caractérisé en ce que**
ladite surface dudit matériau d'humidification (24) est dentelée ou en forme d'étoile.

2. Appareil d'humidification de gaz selon la revendication 1, dans lequel ladite surface dudit matériau d'humidification (24) varie de 15 degrés par rapport à une surface non lisse.

3. Appareil d'humidification de gaz selon la revendication 1, dans lequel ladite surface dudit matériau d'humidification (24) présente des motifs irréguliers.

4. Appareil d'humidification de gaz selon la revendication 1, dans lequel ladite surface dudit matériau d'humidification (24) est maximisée en surface dans l'espace linéaire le plus court possible.

5. Appareil d'humidification de gaz selon la revendication 1, dans lequel ledit dispositif d'humidification comprend en outre un second matériau d'humidification qui est espacé dudit matériau d'humidification (24) et qui absorbe facilement l'humidité et libère facilement l'humidité lorsqu'il est exposé à un environnement sec, où ledit second matériau d'humidification présente une configuration qui génère une turbulence pour un gaz qui devrait passer sur une surface de ladite seconde surface d'humidification.

6. Appareil d'humidification de gaz selon la revendication 5, comprenant en outre un espaceur interposé entre ledit matériau d'humidification (24) et ledit second matériau d'humidification.

7. Appareil d'humidification de gaz selon la revendication 5, dans lequel ladite surface dudit second matériau d'humidification présente des motifs.

8. Appareil d'humidification de gaz selon la revendication 7, dans lequel ladite surface dudit second matériau d'humidification est hors phase par rapport à ladite surface dudit matériau d'humidification (24).

9. Appareil d'humidification de gaz selon la revendication 1, dans lequel ledit matériau d'humidification (24) comprend un médicament imprégné dedans.

10. Appareil d'humidification de gaz selon l'une des revendications 1 à 9, dans lequel ledit dispositif de chauffage (18) est positionné séparément dudit dispositif d'humidification.

11. Appareil d'humidification de gaz selon la revendication 10, dans lequel ledit dispositif de chauffage (18) est positionné en amont dudit dispositif d'humidification.

12. Appareil d'humidification de gaz selon l'une des revendications 1 à 11, comprenant en outre un noyau (20) dans lequel ledit dispositif de chauffage est inséré.

13. Appareil d'humidification de gaz selon la revendication 12, dans lequel ledit noyau (20) comprend un matériau non conducteur.

14. Appareil d'humidification de gaz selon la revendication 1, comprenant en outre un détecteur d'humidité (34) pour mesurer l'humidité d'un gaz qui s'écoule à l'intérieur dudit appareil d'humidification de gaz.

15. Appareil d'humidification de gaz selon la revendication 1, comprenant en outre un détecteur de température (102) pour mesurer une température d'un gaz qui s'écoule à l'intérieur dudit appareil d'humidification de gaz.

16. Appareil d'humidification de gaz selon la revendication 14, comprenant en outre un détecteur de température (102) pour mesurer une température dudit gaz qui s'écoule à l'intérieur dudit appareil d'humidification de gaz.

17. Appareil d'humidification de gaz selon la revendication 15, dans lequel ledit détecteur de température (102) mesure ladite température dudit gaz d'une manière indirecte.

18. Appareil d'humidification de gaz selon la revendication 1, comprenant en outre un détecteur de température (102) pour mesurer une température d'un gaz qui s'écoule à l'intérieur dudit appareil d'humidification de gaz.

19. Appareil d'humidification de gaz selon la revendication 18, dans lequel ledit détecteur de température (102) mesure ladite température dudit gaz d'une manière indirecte en mesurant une température dudit dispositif de chauffage (18).

20. Appareil d'humidification de gaz selon la revendication 14, comprenant en outre un dispositif de commande connecté audit détecteur d'humidité (34) qui commande l'humidité à l'intérieur dudit appareil d'humidification de gaz sur la base d'un signal provenant dudit détecteur d'humidité (34).

21. Appareil d'humidification de gaz selon la revendication 15, comprenant en outre un dispositif de commande (100) connecté audit détecteur de température (102) qui commande la température à l'intérieur dudit appareil d'humidification de gaz sur la base d'un signal provenant dudit détecteur de température (102).

22. Appareil d'humidification de gaz selon la revendication 16, comprenant en outre un dispositif de commande connecté audit détecteur d'humidité (34) et audit détecteur de température (102) qui commande l'humidité et la température à l'intérieur dudit appareil d'humidification de gaz sur la base des signaux provenant dudit détecteur d'humidité (34) et dudit détecteur de température (32).

23. Appareil d'humidification de gaz selon la revendication 2, comprenant en outre : un orifice (16) en communication fluide avec ledit appareil d'humidification de gaz ; et un cathéter inséré à l'intérieur dudit orifice (16).

24. Appareil d'humidification de gaz selon la revendication 1,
dans lequel ledit matériau d'humidification (24) est placé à l'intérieur d'une enveloppe (26) qui présente une configuration qui génère une turbulence dans ledit gaz lorsqu'il passe sur une surface de ladite enveloppe (26).

25. Appareil d'humidification de gaz selon la revendication 24, comprenant en outre une bobine positionnée de manière adjacente à ladite enveloppe (26) qui force un gaz à voyager à travers ou à l'intérieur de ladite bobine.

26. Appareil d'humidification de gaz selon la revendication 24, dans lequel ladite enveloppe (26) comprend une surface intérieure avec un motif irrégulier.

27. Appareil d'humidification de gaz selon la revendication 24, dans lequel ladite enveloppe (26) comprend une surface intérieure avec une barrière en spirale.

28. Appareil d'humidification de gaz selon la revendication 24, dans lequel ledit matériau d'humidification (24) est de forme cylindrique.

29. Appareil d'humidification de gaz selon la revendication 24, dans lequel ledit matériau d'humidification (24) comprend un médicament imprégné à l'intérieur.

30. Appareil d'humidification de gaz selon la revendication 1, dans lequel ledit logement de dispositif de chauffage (217) comprend une pluralité d'ouvertures (219), ladite pluralité d'ouvertures (219) ayant une configuration qui génère une turbulence dans ledit gaz lorsqu'il passe à travers ladite pluralité d'ouvertures (219).

31. Appareil d'humidification de gaz selon la revendication 25, dans lequel ledit dispositif d'humidification comprend en outre un second matériau d'humidification qui est espacé dudit matériau d'humidification (24) et absorbe facilement l'humidité et libère facilement l'humidité lorsqu'il est exposé à un environnement sec, où ledit second matériau d'humidification présente une configuration qui génère une turbulence pour un gaz qui devrait passer sur une surface de ladite seconde surface d'humidification.

32. Appareil d'humidification de gaz selon la revendication 31, comprenant en outre un espaceur interposé entre ledit matériau d'humidification (24) et ledit second matériau d'humidification.

33. Appareil d'humidification de gaz selon la revendication 31, dans lequel ladite surface dudit second matériau d'humidification présente des motifs.

34. Appareil d'humidification de gaz selon la revendication 33, dans lequel ladite surface dudit second matériau d'humidification est hors phase par rapport à ladite surface dudit matériau d'humidification (24).

35. Appareil d'humidification de gaz selon la revendication 25, dans lequel ledit matériau d'humidification (24) comprend un médicament imprégné à l'intérieur.

36. Appareil d'humidification de gaz selon la revendication 25, comprenant en outre un détecteur d'humidité (34) pour mesurer l'humidité d'un gaz qui s'écoule à l'intérieur dudit appareil d'humidification de gaz.

37. Appareil d'humidification de gaz selon la revendication 25, comprenant en outre un détecteur de température (102) pour mesurer une température d'un gaz qui s'écoule à l'intérieur dudit appareil d'humidification de gaz.

38. Appareil d'humidification de gaz selon la revendication 36, comprenant en outre un détecteur de température (102) pour mesurer une température dudit gaz qui s'écoule à l'intérieur dudit appareil d'humidification de gaz.

39. Appareil d'humidification de gaz selon la revendication 37, dans lequel ledit détecteur de température (102) mesure ladite température dudit gaz d'une manière indirecte.

40. Appareil d'humidification de gaz selon la revendication 36, comprenant en outre un dispositif de commande connecté audit détecteur d'humidité (34) qui commande l'humidité à l'intérieur dudit appareil d'humidification de gaz sur la base d'un signal provenant dudit détecteur d'humidité (34).

41. Appareil d'humidification de gaz selon la revendication 37, comprenant en outre un dispositif de commande (100) connecté audit détecteur de température (102) qui commande la température à l'intérieur dudit appareil d'humidification de gaz sur la base d'un signal provenant dudit détecteur de température (102).

42. Appareil d'humidification de gaz selon la revendication 38, comprenant en outre un dispositif de commande (100) connecté audit détecteur d'humidité (34) et audit détecteur de température (102) qui commande l'humidité et la température à l'intérieur dudit appareil d'humidification de gaz sur la base des signaux provenant dudit détecteur d'humidité (34) et dudit détecteur de température (102).

43. Appareil d'humidification de gaz selon la revendication 25, comprenant en outre : un orifice (16) en communication fluide avec ledit appareil d'humidification de gaz ; et un cathéter inséré à l'intérieur dudit orifice (16).

44. Appareil d'humidification de gaz selon la revendication 1, comprenant en outre : un orifice (16) en communication fluide avec ledit appareil d'humidification de gaz ; et un cathéter inséré à l'intérieur dudit orifice (16).
